(19) Europäisches Patentamt
European Patent Office
Office européen des brevets

(11) Veröffentlichungsnummer: **0 443 156 A2**

# (12) EUROPÄISCHE PATENTANMELDUNG

(21) Anmeldenummer: **90124500.1**

(22) Anmeldetag: **18.12.90**

(51) Int. Cl.$^5$: **C07D 333/48**, C07D 333/72, C07D 333/08, C07D 409/14, C08G 61/12, H01B 1/12

(30) Priorität: **22.02.90 DE 4005648**

(43) Veröffentlichungstag der Anmeldung: **28.08.91 Patentblatt 91/35**

(84) Benannte Vertragsstaaten: **BE CH DE FR GB IT LI NL**

(71) Anmelder: **WACKER-CHEMIE GMBH**
**Prinzregentenstrasse 22**
**W-8000 München 22(DE)**

(72) Erfinder: **Hanack, Michael, Prof.Dr.**
**Panoramastrasse 39**
**W-7400 Tübingen 7(DE)**
Erfinder: **Hieber, Gunter, Dipl.-Chem.**
**Adalberger Strasse 74**
**W-7060 Schordorf-Oberberken(DE)**

## (54) Heteroarylenmethine und daraus hergestellte Polymere.

(57) Die vorliegende Erfindung betrifft Verbindungen der Formel (I):

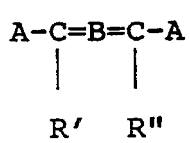

(I),

worin

**A** gleiche oder verschiedene, gegebenenfalls substituierte Heteroarylreste,

**B** gegebenenfalls substituierte Dihydroheteroarendiyliden-Reste oder die aus diesen Resten durch Oxidation an mindestens einem Heteroatom erhältlichen Reste,

**R' und R"** gleiche oder verschiedene Reste, nämlich Wasserstoffatome, $C_1$- bis $C_4$-Alkylreste oder Reste **A** oder Cyanoreste, Halogenatome, Reste der Formel -COOR, oder -OR, worin R ein $C_1$- bis $C_4$-Alkylrest ist.

bedeuten,
Verfahren zur Herstellung dieser Verbindungen und daraus herstellbare Polymere.

EP 0 443 156 A2

Die vorliegende Erfindung betrifft Heteroarylenmethine, Verfahren zu deren Herstellung und daraus hergestellte Polymere.

## Stand der Technik

Heteroarylenmethine sind grundsätzlich bekannt. Die Herstellung von Poly(hetero)arylenmethinen aus Trichlormethylfurfural oder $\alpha,\alpha,\alpha,\alpha',\alpha'$-Pentachlor-p-xylol durch Umsetzung mit (Hetero)aromaten ist in US-A-4 729 851 (Ausgegeben am 8. März 1988, H. Bräunling et al., Wacker-Chemie GmbH) beschrieben. Heteroarylenmethine bestehend aus drei, durch mit jeweils gegebenfalls substituierten Phenylresten substituierte Methinbrücken verbundene Heterocyclen sind bekannt durch A.O. Patil und F. Wudl ( Macromolecules 21, Seite 540 - 542, 1988) und A. Ulman und J. Manassen (Journal of the Chemical Society, Perkin I, Seite 1066-1069, 1979).

## Aufgabe

Aufgabe der vorliegenden Erfindung war es, neue Ausgangsstoffe für Polymere, insbesondere für leitfähige Polymere, bereitzustellen. Ferner war es Aufgabe der vorliegenden Erfindung, neue Polymere zur Verfügung zu stellen. Des weiteren war es Aufgabe der vorliegenden Erfindung, neue Verfahren zur Herstellung von Ausgangsstoffen für Polymere zu entwickeln.

## Beschreibung

Die vorstehend genannten Aufgaben werden von der vorliegenden Erfindung gelöst durch Verbindungen der Formel (I):

$$A-\underset{R'}{\underset{|}{C}}=B=\underset{R''}{\underset{|}{C}}-A \qquad (I),$$

worin

- **A** gleiche oder verschiedene, gegebenenfalls substituierte Heteroarylreste,
- **B** gegebenenfalls substituierte Dihydroheteroarendiyliden-Reste oder die aus diesen Resten durch Oxidation an mindestens einem Heteroarom erhältlichen Reste,

**R'** und **R"** gleiche oder verschiedene Reste, nämlich Wasserstoffatome, $C_1$- bis $C_4$-Alkylreste oder Reste **A** bedeuten, oder Cyanoreste, Halogenatome, Reste der Formel -COOR, oder -OR, worin R ein $C_1$- bis $C_4$-Alkylrest ist.

Vorzugsweise sind die Reste **R'** und **R"** gleiche oder verschiedene Reste, nämlich Wasserstoffatome, $C_1$- bis $C_4$-Alkylreste oder Reste **A**.

Vorzugsweise sind die Reste **A** gegebenenfalls substituierte, gegebenenfalls benzoannelierte Fünfringheteroarylreste. Vorzugsweise sind die Reste **B** gegebenenfalls substituierte, gegebenfalls benzoannelierte Fünfringdihydroheteroarendiylidenreste bzw. die entsprechenden N-Oxide, Sulfoxide und/oder Sulfone. Als Substituenten für die Reste **A** bzw. **B** sind Halogenatome und gegebenenfalls halogenierte $C_1$- bis $C_4$-Alkylreste bevorzugt, insbesondere Fluor-, Chlor- und Bromatome. Es ist bevorzugt, daß die Reste **R'** und **R"** Wasserstoffatome oder Methylgruppen sind oder jeweils eine der für die Reste **A** angegebenen Bedeutungen haben. Es ist besonders bevorzugt, daß die Reste **R'** und **R"** gleich sind und speziell, daß sie jeweils Wasserstoffatome sind oder jeweils gleiche Reste **A** bedeuten.

Beispiele für unsubstituierte, gegebenenfalls benzoannelierte Fünfringheteroarylreste **A** sind Thiophenyl-, Benzothiophenyl-, Furanyl-, Benzofuranyl-, Pyrrolyl-, Indolyl-, Thiazolyl-, Thianaphthenyl-, Pyrazolyl-, Benzopyrazolyl-, Imidazolyl-, Benzimidazolyl-, Oxazolyl-, Benzoxazolyl-, Triazolyl-, Benzotriazolylreste.

Beispiele für unsubstituierte, gegebenenfalls benzoannelierte Fünfringdihydroheteroarendiylidenreste bzw. die aus diesen Resten durch Oxidation an mindestens einem Heteroatom erhältlichen Reste **B** sind Dihydrothiophendiylidenreste, wie der Dihydrothiophen-2,5-diylidenrest, sowie dessen Sulfoxid, Dihydrobenzothiophendiylidenreste und deren Sulfoxide, 3-Sulfolendiyliden-, Benzosulfolendiyliden-, Dihydropyrrol-2,5-diyliden, Dihydroindol-2,3-diyliden-, Isoindolin-1,3-diyliden-, Dihydrofurandiylidenreste. Dihydrobenzofurandiylidenreste, Dihydrimidazoldiylidenreste. Dihydrobenzimidazoldiylidenreste, Dihydrooxazoldiylidenreste, Dihydrobenzoxazoldiylidenreste, Dihydrotriazoldiylidenreste, Dihydrobenzotriazoldiylidenreste.

Als unsubstituierte, gegebenenfalls benzoannelierte Fünfringdihydroheteroarendiylidenreste bzw. die aus diesen Resten durch Oxidation an mindestens einem Heteroatom erhältlichen Reste **B** sind aber auch solche Reste anzusehen, die mit mehr als einem Benzolring anneliert sind. Dies sind beispielsweise Reste der Formel (XXIII):

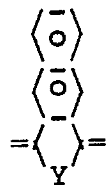

(XXIII),

und solche der Formel (XXIV):

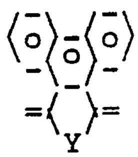

(XXIV),

wobei in den obigen Formeln (XXIII) und (XXIV)

**Y** ein Sauerstoff- oder Schwefelatom, ein Rest der Formel SO, $SO_2$, N-H, N-O oder N-R ist, wobei

**R** für einen $C_1$-bis $C_4$-Alkylrest steht.

Bevorzugt unter den Resten **B** sind solche mit nur einem Heteroatom im Ring. Bevorzugt als benzoannelierte Reste **B** sind die entsprechenden benzo[c]-fünfringheterocylischen Reste mit einem Heteroatom im Ring, also Reste **B**, bei denen die Benzoannelierung in 3,4-Position relativ zum Heteroatom ( = Postion 1) vorliegt.

Weitere Beispiele für bevorzugte unsubstituierte benzoannelierte Reste **B** sind Reste der Formel (II):

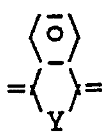

(II);

Beispiele für bevorzugte unsubstituierte nicht benzoannelierte Reste **B** sind Reste der Formel (III):

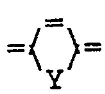

(III),

wobei in den oben aufgeführten Formel (II) und (III) der Rest

**Y** ein Sauerstoff- oder Schwefelatom, ein Rest der Formel SO, $SO_2$, N-H, N-O oder N-R ist, wobei

**R** für einen $C_1$-bis $C_4$-Alkylrest steht.

Bevorzugt als Substituenten der Reste **A** und **B** sind Halogenatome, Pseudohalogenreste, wie der Cyanorest, gegebenenfalls halogenierte $C_1$- bis $C_6$-Kohlenwasserstoffreste und gegebenenfalls halogenierte $C_1$- bis $C_6$- Kohlenwasserstoffoxireste.

Beispiele für Substituenten der Reste **A** und **B** sind Fluor-, Chlor-, Brom- und Jodatome, Cyanoreste, Methyl-, Ethyl-, n-Propyl-, Isopropyl-, n-Butyl-, sec.-Butyl-, t.-Butyl-, Chlormethyl-, Trichlormethyl-, Trifluormethyl-, 3,3,3-Trifluor-propylgruppen.

Beispiele für besonders bevorzugte Verbindungen der Formel (I) sind solche der Formel (IV), (V) oder (VI):

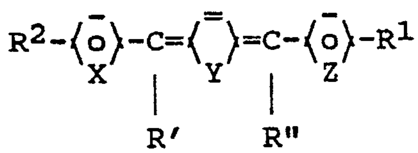

(IV),

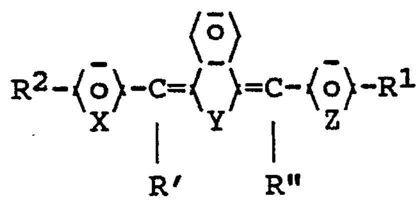

(V),

bzw.

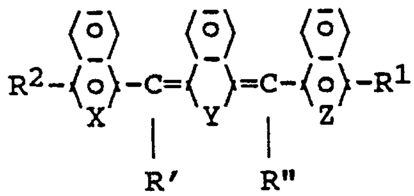

(VI),

worin

**X und Z** gleiche oder verschiedene zweiwertige Reste bedeuten, nämlich Sauerstoff- oder Schwefelatome, Reste der Formel N-H oder N-R,

**$R^1$ und $R^2$** Wasserstoffatome, Halogenatome, Pseudohalogenreste, wie der Cyanorest, gegebenenfalls halogenierte $C_1$- bis $C_6$-Kohlenwasserstoffreste und gegebenenfalls halogenierte $C_1$- bis $C_6$- Kohlenwasserstoffoxireste,

**Y** und **R** die unter den Formeln (II) und (III), und

**R'** und **R"** die unter der Formel (I)

angegebenen Bedeutungen haben.

Von den oben aufgeführten Verbindungen der Formeln (IV), (V) und (VI) sind besonders die der Formeln (IV) und (V) bevorzugt.

Vorteile der erfindungsgemäßen Sulfone (**Y** = $SO_2$) ist ihre hohe thermische Stabilität (200 °C bis 250 °C) und ihre Stabilität gegenüber Sauerstoff.

Verfahren

Alle in den nachstehend geschilderten Verfahren 1, Verfahren 2 und Verfahren zur Herstellung und Verarbeitung von Polymeren als Reagentien, Hilfsstoffe, Lösungsmittel, Katalysatoren, Dotierungsmittel, Schutzgase, Polymere, weitere Monomere und alle anderen verwendbaren Stoffe können einzeln oder im Gemisch eingesetzt werden.

Verfahren 1:

Die Verbindungen der Formel (I) können hergestellt werden durch Umsetzung von Dihydroheteroarenen der Formel (VII)

$BH_4$ (VII),

mit Verbindungen der Formeln (VIII) und (IX)

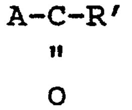

(VIII)

bzw.

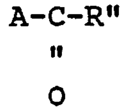

(IX)

wobei in den obigen Formeln (VII), (VIII) und (IX) **A**, **B**, **R'** und **R"** die obenstehend unter Formel (I) angegebenen Bedeutungen haben.

Es kann je eine Verbindung der Formeln (VII), (VIII) und (IX), es können auch Gemische aus mindestens zwei Verbindungen der Formel (VII), aus mindestens zwei Verbindungen der Formel (VIII) und/oder aus mindestens zwei Verbindungen der Formel (IX) eingesetzt werden.

Vorzugsweise werden nach dem oben skizzierten Verfahren Verbindungen der Formel (I) hergestellt, für die

**R'** und **R"** gleich sind, insbesondere Wasserstoffatome oder gleiche Reste **A** und speziell Wasserstoffatome bedeuten.

Vorzugsweise werden als Verbindungen der Formel (VII) solche der Formel (X) eingesetzt:

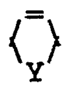

(X),

und/oder Verbindungen der Formel (XI)

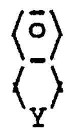

(XI),

und/oder Verbindungen der Formel (XXVII)

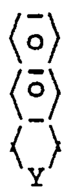

(XXVII);

und/oder Verbindungen der Formel (XXVIII)

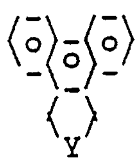

(XXVIII);

worin **Y** jeweils die oben unter der Formel (II) angegebene Bedeutung hat.

Es kann eine Verbindung der Formel (X), (XI), (XXVII) oder (XXVIII) im erfindungsgemäßen Verfahren eingesetzt werden, es kann auch ein Gemisch aus Verbindungen der Formel (X), aus Verbindungen der Formel (XI) oder aus mindestens einer Verbindung der Formel (X) mit mindestens einer Verbindung der Formel (XI) eingesetzt werden. Vorzugsweise wird nur eine Verbindung eingesetzt, also eine Verbindung der Formel (X) oder eine Verbindung der Formel (XI).

Als Verbindungen der Formel (VIII) werden im erfindungsgemäßen Verfahren vorzugsweise Verbindungen der nachstehenden Formeln (XII) oder (XIII) eingesetzt; Als Verbindungen der Formel (IX) werden im erfindungsgemäßen Verfahren vorzugsweise Verbindungen der nachstehenden Formeln (XIV) oder (XV) eingesetzt:

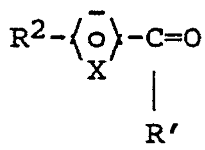 (XII), 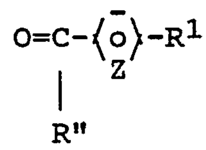 (XIII),

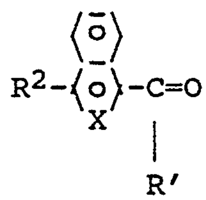 (XIV) bzw. 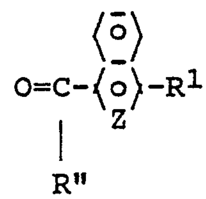 (XV),

worin **R¹**, **R²**, **R'**, **R''**, **X** und **Z** die oben unter den Formeln (IV) bis (VI) angegebenen Bedeutungen haben.

Falls Y im Produkt gemäß Formeln (IV), (V) oder (VI) ein Schwefelatom bedeuten soll, so werden in einer bevorzugten Ausführungsform des erfindungsgemäßen Verfahrens Verbindungen der Formel (X) und/oder (XI) eingesetzt, für die Y einen Rest der Formel SO bedeutet, und das als Reaktionsprodukt erhaltene Sulfoxid anschließend reduziert.

Es kann von Vorteil sein, eine Komponente im Überschuß bzw. Unterschuß einzusetzen, sei es, um die Reaktion zu beschleunigen, sei es, um mögliche Nebenreaktionen zu unterdrücken. Im erfindungsgemäßen Verfahren werden jedoch Verbindungen der Formel (VII) zur Summe der Verbindungen der Formeln (VIII) und (IX) vorzugsweise in einem Molverhältnis von 0,1 zu 1 bis 10 zu 1, insbesondere von 0,3 zu 1 bis 0,75 zu 1, speziell von 0,4 zu 1 bis 0,6 zu 1, eingesetzt.

Das erfindungsgemäße Verfahren wird vorzugsweise in Gegenwart einer Base, insbesondere einer starken Base, durchgeführt. Bevorzugte Basen sind u.a. **Alkalimetall- und Erdalkalimetallhydroxide**, wie LiOH, NaOH, KOH, RbOH, CsOH, $Mg(OH)_2$, $Ca(OH)_2$, $Sr(OH)_2$, $Ba(OH)_2$; **Alkalimetall- und Erdalkalimetallalkoholate**, insbesondere die Natrium- und Kaliumsalze von $C_1$- $C_{18}$-Alkanolen, wie Natriummethylat, Natriumethylat, Kaliumethylat und Natriumisopropylat; **Alkalimetall- und Erdalkalimetallamide**, insbesondere solche von Ammoniak, von Alkyl- und Dialkylaminen mit jeweils 1 bis 18C-Atomen pro Alkylrest, wie $LiNH_2$, $NaNH_2$, $KNH_2$, Lithiumdimethylamid, Lithiumdiethylamid und Lithiumdiisopropylamid; **Alkalimetall- und Erdalkalimetallhydride**, wie NaH, KH und $CaH_2$; Alkali- und Erdalkalimetallalkyle, wie n-Butyllithium und t-Butyllithium; **Amine**, wie Mono- und Dialkylamine, Pyridine, Pyrimidine und Morpholine.

Im erfindungsgemäßen Verfahren wird Base, falls eingesetzt, vorzugsweise in Mengen von 5 Mol% bis 1000 Mol%, insbesondere von 10 Mol% bis 100 Mol%, jeweils bezogen auf die Summe der Molzahlen von eingesetzter Verbindung der Formeln (VIII) und (IX), angewandt.

Das erfindungsgemäße Verfahren wird vorzugsweise bei Temperaturen von 0 °C bis 250 °C, insbesondere von 15 °C bis 50 °C durchgeführt.

Das erfindungsgemäße Verfahren kann bei Drücken weit über und weit unter 0,1 MPa (abs.) durchgeführt werden; bevorzugt sind Drücke von 0,09 bis 0,11 MPa (abs.), insbesondere der Druck der umgebenden Atmosphäre, also etwa 0,101 MPa (abs.).

Das erfindungsgemäße Verfahren kann in Anwesenheit oder in Abwesenheit von Lösungsmitteln durchgeführt werden. Vorzugsweise wird es in Anwesenheit von Lösungsmittel durchgeführt. Falls Lösungsmittel verwendet werden, sind Lösungsmittel oder Lösungsmittelgemische mit einem Siedepunkt bzw. Siedebereich von bis zu 120° C bei 0,1 MPa bevorzugt. Beispiele für solche Lösungsmittel sind Wasser; Alkohole, wie Methanol, Ethanol, n-Propanol, iso-Propanol; Ether, wie Dioxan, Tetrahydrofuran, Diethylether, Diethylenglycoldimethylether; chlorierte Kohlenwasserstoffe, wie Dichlormethan, Trichlormethan, Tetrachlormethan, 1,2-Dichlorethan, Trichlorethylen; Kohlenwasserstoffe, wie Pentan, n-Hexan, Hexan-Isomerengemische, Heptan, Oktan, Waschbenzin, Petrolether, Benzol, Toluol, Xylole; Schwefelkohlenstoff und Nitrobenzol, oder Gemische dieser Lösungsmittel.
Weniger bevorzugt als Lösungsmittel sind Ketone, wie Aceton, Methylethylketon, Methyl-isobutylketon, da Ketone mit Verbindungen der Formel (VII) Kondensationsprodukte bilden können.

Die Bezeichnung Lösungsmittel bedeutet nicht, daß sich alle Reaktionskomponenten in diesem lösen müssen. Die Reaktion kann auch in einer Suspension oder Emulsion eines oder mehrerer Reaktionspartner durchgeführt werden. Die Reaktion kann auch in einem Lösungsmittelgemisch mit einer Mischungslücke ausgeführt werden, wobei in jeder der Mischphasen jeweils mindestens ein Reaktionspartner löslich ist.

Nach der Reaktion wird Base, falls verwendet, bzw. deren Reaktionsprodukte und Lösungsmittel, falls verwendet, vorzugsweise abgetrennt. Basen und deren Reaktionsprodukte können beispielsweise durch

Chromatographie an anorganischen Sorbentien, wie Kieselgel oder Aluminiumoxid, oder an einem Ionenaustauscher abgetrennt werden. Die Reaktionsmischung wird vorzugsweise destillativ von Lösungsmittel, falls verwendet, befreit. Insbesondere wird das die erfindungsgemäßen Produkte enthaltende Reaktionsgemisch durch Filtration über Kieselgel und anschließender destillativer Entfernung des Lösungsmittel (sofern verwendet) gereinigt. Die Produkte sind im allgemeinen kristallin und können, falls ein Produkt hoher Reinheit gewünscht wird, durch Umkristallisation weiter gereinigt werden.

Falls Y im Produkt gemäß Formeln (IV), (V) oder (VI) ein Schwefelatom bedeuten soll, so werden in einer bevorzugten Ausführungsform des erfindungsgemäßen Verfahrens Verbindungen der Formel (X) und/oder (XI) eingesetzt, für die Y einen Rest der Formel SO bedeutet, und das als Reaktionsprodukt erhaltene Sulfoxid anschließend reduziert. Eine analoge Reaktion ist von D.W. Chasar und T.M. Pratt (Synthesis 1976, Seite 262) beschrieben. Als Reduktionsmittel hierfür eignen sich insbesondere $LiAlH_4$, HJ, $NaBH_4$, $TiCl_2$, $PCl_3$, Tributylstannan, $H_2$/Pd, Triphenylphosphin, Stannan, Acetylchlorid, $CH_3SiCl_3{}^*NaJ$, t-Butylbromid, Tris(dimethylamino)phosphin$^*J_2$, Phosphorigsäure-(O-phenylen-ester)-chlorid.

Verfahren 2:

Verbindungen der Formel (I) können auch hergestellt werden, indem Verbindungen (VIII) und/oder (IX) mit Lithiumsalzen der den Resten **B** in Formel (I) entsprechenden Heteroarenen (kurz: Lithiumverbindung) zur Reaktion gebracht werden und die so erhaltenen Produkte anschließend reduziert, vorzugsweise mit Jodwasserstoff und/oder dessen Salzen, insbesondere mit Jodwasserstoff, umgesetzt werden. Die Reduktion gelingt jedoch u.a. auch mit HCOOH, mit weiteren Jodreagentien, wie $MgI_2$/Mg, mit Trialkyljodsilan oder einem Komplex aus Trialkylchlorsilan mit Alkalimetalljodid. Bei Reaktion mit Jodreagentien werden i.a. die Hydroxylgruppen des primär entstandenen bis-Carbinols durch Jod substituiert, worauf sich - meistens spontan - Jod abspaltet. Das Jod kann, falls erwünscht, durch geeignete Reagentien, wie Natriumdithionit, abgefangen werden.

Dieses Verfahren empfiehlt sich besonders, wenn im gewünschten Produkt der Formel (I) R' und R" jeweils Reste A, also gegebenenfalls substituierte Heteroarylreste, bedeuten. Das erfindungsgemäße Verfahren 2 wird vorzugsweise bei Temperaturen von 0 °C bis 100 °C, insbesondere von 15 °C bis 50 °C durchgeführt.

Das erfindungsgemäße Verfahren 2 kann bei Drücken weit über und weit unter 0,1 MPa (abs.) durchgeführt werden; bevorzugt sind Drücke von 0,09 bis 0,11 MPa (abs.), insbesondere der Druck der umgebenden Atmosphäre, also etwa 0,101 MPa (abs.).

Das erfindungsgemäße Verfahren 2 kann in Anwesenheit oder in Abwesenheit von Lösungsmitteln durchgeführt werden. Vorzugsweise wird es in Anwesenheit von Lösungsmittel durchgeführt. Falls Lösungsmittel verwendet werden, sind Lösungsmittel oder Lösungsmittelgemische mit einem Siedepunkt bzw. Siedebereich von bis zu 120° C bei 0,1 MPa bevorzugt. Beispiele für solche Lösungsmittel sind die für das Verfahren 1 angegebenen Lösungsmittel und Lösungsmittelgemische.

Es kann von Vorteil sein, eine Komponente im Überschuß bzw. Unterschuß einzusetzen, sei es, um die Reaktion zu beschleunigen, sei es, um mögliche Nebenreaktionen zu unterdrücken. Im erfindungsgemäßen Verfahren 2 werden jedoch Lithiumverbindung zur Summe der Verbindungen der Formeln (VIII) und (IX) vorzugsweise in einem Molverhältnis von 0,1 zu 1 bis 10 zu 1, insbesondere von 0,3 zu 1 bis 0,75 zu 1, speziell von 0,4 zu 1 bis 0,6 zu 1, eingesetzt.

Im erfindungsgemäßen Verfahren 2 bildet sich primär das entsprechende Bis-Carbinol, welches beispielsweise mit Jodwasserstoff oder dessen Salzen zur Bis-Jodverbindung umgesetzt wird, die wiederum spontan Jod abspaltet. Vorzugsweise wird dieses sich bildende Jod im Verfahren 2 durch ein Reduktionsmittel, beispielsweise Natriumdithionit, abgefangen.

Vorzugsweise werden als Lithiumsalze der den Resten **B** in Formel (I) entsprechenden Heteroarenen die entsprechenden Dilithiumsalze eingesetzt, insbesondere solche der Formeln (XVI), (XVII), (XXV) bzw. (XXVI):

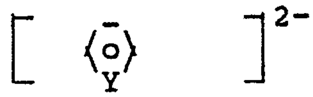 $(Li^+)_2$ (XVI),

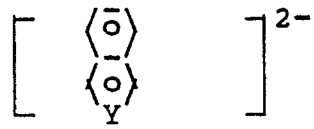 $(Li^+)_2$ (XVII),

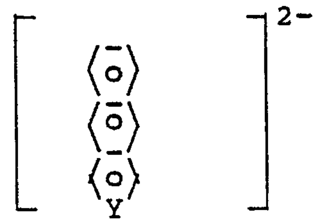 $(Li^+)_2$ (XXV),

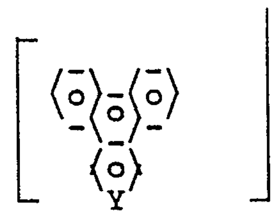 $(Li^+)_2$ (XXVI),

worin **Y** jeweils die oben unter der Formel (II) angegebene Bedeutung hat.

Als Verbindungen der Formeln (VIII) und oder (IX) werden in dem erfindungsgemäßen Verfahren 2 vorzugsweise solche der Formel (XVIII)

$A_2C=O$ (XVIII)

eingesetzt, worin die Reste A die unter der Formel (I) angegebenen Bedeutungen haben und insbesondere gleiche Reste sind.

Polymere

Die Verbindungen der Formel (I) eignen sich zur Herstellung von Polymeren, insbesondere zur Herstellung von Polymeren mit konjugierten Doppelbindungen.

Es sind dies insbesondere Polymere der Formel (XIX):

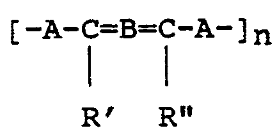 (XIX),

worin

**n** eine ganze Zahl bedeutet und

**A**, **B**, **R'** und **R"** die unter Formel (I) angegebenen Bedeutungen haben.

Vorzugsweise sind dies Polymere der Formeln (XX), (XXI) bzw.(XXII):

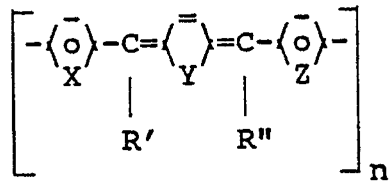

(XX),

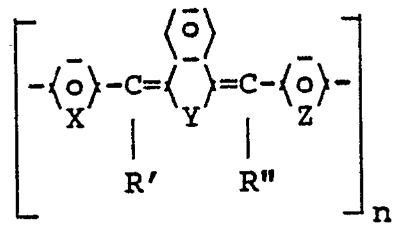

(XXI),

bzw.

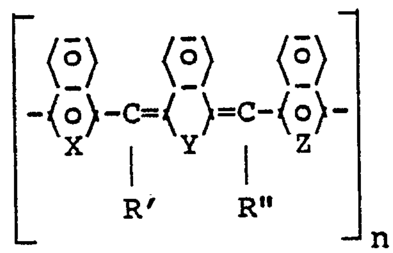

(XXII),

worin

**X** und **Z** gleiche oder verschiedene zweiwertige Reste bedeuten, nämlich Sauerstoff- oder Schwefelatome, Reste der Formel N-H oder N-R,

**n** die unter Formel (XIX),

**Y** und **R** die unter den Formeln (II) und (III), und

**R'** und **R''** die unter der Formel (I)

angegebenen Bedeutungen haben.

Die Reste **R'** und **R''** sollten nicht zu sterisch anspruchsvoll sein, damit die Konjugation im Polymer nicht gestört ist.

Vorzugsweise sind die Reste **R'** und **R''** jeweils Wasserstoffatome.

Die Zahl **n** beträgt vorzugsweise mindestens 10, insbesondere mindestens 50.

Die vorliegende Erfindung betrifft auch Komplexe von Polymeren der Formeln (XIX), (XX), (XXI) und (XXII) mit Dotierungsmitteln. Diese Komplexe können elektrisch geladen sein.

Die Darstellung der Polymere der Formeln (XIX), (XX), (XXI) bzw. (XXII) kann durch übliche chemische und elektrochemische Behandlung der Verbindungen der Formeln (I), (IV), (V) bzw. (VI) erfolgen.

Die chemische Polymerisation kann in Gegenwart vieler üblicher Oxidantien, beispielsweise durch Umsetzung mit Nitrosyltetrafluoroborat erfolgen, insbesondere für Verbindungen der Formeln (IV), (V) oder (VI), für die $R^1$ und $R^2$ jeweils Wasserstoffatome bedeuten. Verbindungen der Formeln (IV), (V) oder (VI), für die $R^1$ und $R^2$ jeweils Halogenatome bedeuten, insbesondere Chlor- und/oder Bromatome, können u.a. mit metallorganischen Verbindungen, insbesondere n-Butyllithium, polymerisiert werden.

Die elektrochemische Polymerisation wird vorzugsweise in Gegenwart eines Leitsalzes bzw. Dotierungsmittels durchgeführt.

Beispiele für Dotierungsmittel, mit denen die erfindungsgemäßen Polymere versetzt sein können, sind Alkalimetalle, wie Natrium oder Kalium; Protonensäuren wie $H_2SO_4$, $HClO_4$, $H_2Cr_2O_7$, HJ und $HNO_3$; Lewis-Säuren wie $SbCl_5$, $AsCl_5$, $TiCl_4$, $FeCl_3$, $SnCl_4$, $ZnCl_2$, $AsF_5$ und Halogen, wie z.B. Jod. Es kann ein Dotierungsmittel, es können aber mindestens zwei Dotierungsmittel als Gemisch oder zeitlich hintereinander verwendet werden. Die dotierten Polymere enthalten vorzugsweise von 0 bis 50, besonders vorzugsweise von 0,01 bis 30, insbesondere von 0,1 bis 20 Gew.-% Dotierungsmittel.

Dotierungsmittel kann bzw. können, falls verwendet, vor, während und/oder nach der Polymerisation zugesetzt werden. Wird die Dotierung nach der Polymerisation durchgeführt, so wird Dotierungsmittel vorzugsweise aufgebracht, indem man die Dämpfe oder Lösungen des Dotierungsmittels auf die Polymeren einwirken läßt. Meist arbeitet man bei etwa 10 bis 30 °C, meist unter Feuchtigkeitsausschluß, oft unter Luftausschluß.

Die Polymerisation wird vorzugsweise in Gegenwart eines Lösungsmittels oder eines Lösungsmittelgemisches durchgeführt. Als Lösungsmittel bzw. Lösungsmittelgemisch sind die für Verfahren 1 als bevorzugt aufgeführten Lösungsmittel einzeln oder im Gemisch bevorzugt. Je nach den zur Polymerisation benutzten Reagentien kann es ratsam sein, wasserfreie oder aprotische Lösungsmittel zu verwenden. Gegebenenfalls kann die Durchführung der Polymerisation unter Schutzgas, wie Stickstoff oder Argon, vorteilhaft sein.

Es kann von Vorteil sein, die erfindungsgemäße Polymerisation in Gegenwart mindestens eines weiteren Monomeren oder des bzw. der entsprechenden Polymeren durchzuführen. Verbindung der Formel (I) und mindestens ein weiteres Monomer können gleichzeitig oder in beliebiger Reihenfolge nacheinander polymerisiert werden. Besonders bevorzugt ist die Polymerisation von mindestens einer Verbindung der Formel (I) in einer Matrix aus feinteiligem Polymer, in einer Lösung von Polymer und/oder in einer Matrix von in Lösungsmittel gequollenem Polymer. Solch ein Verfahren ist in DE-A-38 29 753 (angemeldet am 1. September 1988, R. Becker et al., Wacker-Chemie GmbH) bzw. in der prioritätsgleichen US-Anmeldung (Serial No. 397 590, angemeldet am 23. August 1989) beschrieben. Solche modifizierte Polymere lassen sich, insbesondere wenn das weitere Polymer ein Thermoplast ist, mühelos formen und verarbeiten.

Beispiele für solche geeigneten weiteren Polymere sind organische synthetische Polymere wie Polyvinylchlorid, Polyethylen, Polypropylen, Polyvinylacetat, Polycarbonat, Polyacrylat, Polymethacrylat, Polymethylmethacrylat, Polystyrol, Polyacrylnitril, Polyvinylidenchlorid, Polyvinylfluorid, Polyvinylidenfluorid, Polyvinylidencyanid, Polybutadien, Polyisopren, Polyether, Polyester, Polyamid, Polyimid, Silikone, Polyvinylalkohol, Polyvinylpyrrolidon, Polyacrylamid, Polyethylenglykol und deren Derivate und ähnliche einschließlich Copolymere wie Styrol-Acrylat Copolymere, Vinylacetat-Acrylat Copolymere und Ethylen-Vinylacetat Copolymere sowie natürliche Polymere wie Cellulose, Stärke, Casein und natürliches Gummi, sowie halbsynthetische hochmolekulare Verbindungen wie Cellulosederivate, z. B. Methylcellulose, Hydroxymethylcellulose und Carboxymethylcellulose.

Beispiele für geignete weitere Monomere sind die den oben genannten Polmeren entsprechenden Monomere bzw. Monomergemische.

Die erfindungsgemäßen Polymere sind je nach Art und Umfang ihrer Dotierung **elektrisch leitende oder halbleitende Verbindungen**. Sie können beispielsweise in Batterien, Akkumulatoren, Schaltungen, Schaltern, Elektroden, als bzw. in elektrischen Abschirmungen und elektrochemischen Sensoren, **als antistatische Materialien**, u.a. für Kleidung, für elektrische und elektronische Geräte. Sie eignen sich auch **für Beschichtungen von optischen Artikeln**, wie Linsen, Scheiben, u.a. Fensterscheiben, u.a. zu deren thermischen Isolierung. Sie können auch Verwendung finden **in Wirkstoff enthaltenden Präparaten**, insbesondere auf dem Bereich der Agrikultur und Medizin; so kann ein kontrollierter Transport von Wirkstoffen, wie Pflanzenschutzmitteln, Schädlingsbekämpfungsmitteln und Arzneimitteln erfolgen. Die Freisetzung des Wirkstoffes, beispielsweise aus einem Implantat am oder im menschlichen oder tierischen Körper, kann durch Anlegen einer Steuerspannung geregelt werden. Die erfindungsgemäßen Polymere können gegebenenfalls vermischt mit anderen Polymeren, eingesetzt werden. Bedingt durch ihre Komplexierungsfähigkeit können sie als **Absorbentien für Chemikalien**, u.a. für Schwermetalle bzw. deren Salze, eingesetzt werden. Sie können als **Katalysatoren** oder deren Bestandteil bzw. Vorläufer Verwendung finden, beispielsweise, wenn sie mit einem katalytisch wirksamen Metallsalz komplexiert sind.

Die erfindungsgemäßen Polymere können auch aufgrund ihrer komplexbildenden Eigenschaften als Absorbentien für Schwermetalle und als Katalysatoren verwendet werden.

In den nachfolgenden Beispielen sind, falls jeweils nicht anders angegeben,

a) alle Mengenangaben auf das Gewicht bezogen;

b) alle Drücke 0,10 MPa (abs.);

c) alle Temperaturen 20° C ;

d) alle UV-Spektren in Methanol gemessen, wobei die Hauptabsorptionsbande in Fettdruck wiedergegeben ist und die Wellenlängen in nm angegeben sind;

e) alle $^1$H-NMR in Dimethylsulfoxid-$d_6$ mit Tetramethylsilan (TMS) als innerem Standard gemessen; die chemischen Verschiebungen sind in ppm relativ zu TMS angegeben;

f) alle Ausgangsstoffe käuflich oder nach in der Literatur beschriebenen Verfahren herstellbar;

g) alle Produkte mit Ausnahme der Polymere, mit Hilfe von Flash-Chromatographie aufgearbeitet.

Beispiele

Beispiel 1: Sulfone

(UV- und NMR Spektraldaten sind in Tab. 1 bzw. Tab. 2 angegeben)

1.1 2,5-Bis-(2-thienylmethyliden)-2,5-dihydrothiophen-1,1-dioxid (1a)

($C_{14}H_{10}O_2S_3$, M = 306)

80 mmol = 9.4 g 3-Sulfolen (M = 118) und 170 mmol = 19.04 g = 15.9 ml Thiophen-2-aldehyd (M = 112, d = 1.2 g/cm³) wurden zu einer alkalischen Lösung von 1 g NaOH in 200 ml Ethanol gegeben. Man rührte 3 Tage bei Raumtemperatur.
Der entstehende gelbe Niederschlag wurde abgesaugt und gut mit Ethanol und Wasser gewaschen.
Rohausbeute: 2.5 g

Die Reaktionslösung wird eingeengt und mit Aceton versetzt. Die chromatographische Abtrennung der polymeren Bestandteile erfolgt mit Kieselgel und Aceton als Elutionsmittel. Die Acetonlösung wurde eingeengt und in den Kühlschrank gestellt. Erhalten wurden braungelbe Kristalle.
Rohausbeute: 1 g

Das Rohprodukt wurde zweimal aus Aceton umkristallisiert. Man erhielt organgegelbe Nadeln.
Ausbeute: 2 g = 8.7 % d. Theorie
Smp: 220 ° C.
MS: m/e 306 ($M^+$)

1.2 2,5-Bis(2-(N-Methylpyrryl)-methyliden) 2,5-dihydrothiophen-1,1-dioxid (1b)
($C_{16}H_{16}N_2O_2S$, M = 300)

30 mmol = 3.56 g 3-Sulfolen (M = 118) und 80 mmol = 8.72 g = 8.6 ml N-Methylpyrrol-2-aldehyd (M = 109, d = 1.016 g/cm³) wurden zu 100 ml einer kalt gesättigten Lösung von $NaOCH_3$ in Methanol gegeben. Nach 4 Tagen Kochen unter Rückfluß wurde der entstandene Niederschlag abgesaugt und mit Methanol und Wasser gewaschen.
Rohausbeute: 8 g
Nach zweimaligem Umkristallisieren mit Aceton erhielt man tiefrote Kristalle.
Ausbeute: 4 g = 44.4 % der Theorie
Smp.: 243 ° C
MS: m/e 300 ($M^+$)

1.3 2,5 Bis (2-pyrrylmethyliden)-2,5-dihydrothiophen-1,1-dioxid (1c)
($C_{14}H_{12}N_2O_2S$ , M = 272)

20 mmol = 2,36 g 3-Sulfolen (M = 118) und 42 mmol = 4 g Pyrrol-2-aldehyd (M = 95) werden in einer alkalischen Lösung von 1,5 g NaOH und 1,9 g $NaOCH_3$ in 70 ml Methanol gelöst. Man läßt 5 Tage bei Raumtemperatur rühren. Das schwarzbraune Reaktionsgemisch wird eingeengt.
Die chromatographische Reinigung erfolgt mit Kieselgel/Ether, Kieselgel/Toluol : Ether = 2 : 3 und schließlich mit Aluminiumoxid/Ether. Beim Einengen der etherischen Lösung erhält man einen gelben Niederschlag der abgesaugt und getrocknet wird.
Ausbeute: 80 mg = 1.5 % d. Theorie
Smp.: 174° C (Zersetzung)
MS: m/e 272 ($M^+$)

1.4 2,5 Bis-(2-(5-Brom-thienyl)methyliden)-2,5-dihydrothiophen-1,1-dioxid (1 d)
($C_{14}H_8Br_2O_2S_3$, M = 463.8)

10 mmol = 1.18 g 3-Sulfolen (M = 118) und 22 mmol = 4.2 g = 2.6 ml 5-Brom-thiophen-2-aldehyd (M = 190.9, d = 1.607 g/cm³) wurden zu einer Lösung aus 0.6 g NaOH und 25 ml $H_2O$ und 10 ml Tetrahydrofuran gegeben. Dabei löste sich der Aldehyd nur teilweise; man erhielt 2 Phasen. Nach 3-tägigem Rühren bei Raumtemperatur wurde der entstandene gelbe Niederschlag abgesaugt.
Rohprodukt: 300 mg

Das Reaktionsgemisch wurde eingeengt und mit Essigsäureethylester versetzt. Die chromatographische Reinigung erfolgte zuerst mit Kieselgel/Essigester und dann mit $Al_2O_3$/ Toluol bzw. Kieselgel/Toluol. Die Toluol - Lösung wurde eingeengt und in den Kühlschrank gestellt. Man erhielt gelbe Kristalle Rohprodukt: 500 mg

Durch zweimaliges Umkristallisieren aus Aceton erhielt man gelbe Kristalle.
Ausbeute: 500 mg = 10.8 % d. Theorie
Smp.: 249 - 50° C
MS: m/e 462 ($M^+$)

1.5 1,3-Bis-(arylmethyliden)-1,3-dihydroisothianaphthen-2,2-dioxid (3)

10 mmol = 1.68 g 1,3-Dihydroisothianaphthen-2,2-dioxid (M = 168) (2) und 22 mmol Aryl-2-aldehyd wurden zu 70 ml einer kalt gesättigten Lösung von $NaOCH_3$ in Methanol gegeben. Nach 3 Tagen Kochen unter Rückfluß wurde der entstandene Niederschlag abgesaugt und mit viel Methanol gewaschen. Umkristallisiert wurde aus Aceton.

aryl = 2-thienyl (3 a) ($C_{18}H_{12}O_2S_3$, M = 356)
gelbe Kristalle
Ausbeute: 600 mg = 16.9 % d. Theorie
Smp.: 223° C
MS: m/e 356 ($M^+$)

aryl = 2(N-Methylpyrryl (3 b) ($C_{20}H_{18}N_2O_2S$, M = 350)
gelbe nadelförmige Kristalle
Ausbeute: 1.5 g = 42.8 % d. Theorie
Smp.: 298° C
MS: m/e 350 ($M^+$)

aryl = 2-pyrryl (3 c) ($C_{18}H_{14}N_2O_2S$, M = 322)
Die Synthese erfolgte analog. Nach der chromatographischen Reinigung mit Kieselgel/Essigester und Kieselgel/Toluol kristallisierte das Produkt aus Toluol in gelben Kristallen.
Ausbeute: 1.1 g = 34.2 % d. Theorie
Smp.: 228° C (Zersetzung)
MS: m/e 322 ($M^+$)

Tab. 1

| UV Absorption in (nm) | | | | | | |
|---|---|---|---|---|---|---|
| 1 a | 416 sh | 392.4 | 355 | 323 | 265.6 | |
| 1 b | 430 | | | 343 | 285 | 211 |
| 1 c | 418 | | 369 | 337 | 280 | 217 |
| 1 d | 429 sh | 408 | 388 sh | 330 | 276 | 209 |
| 3 a | 368 | 357 sh | 335 | 323 sh | 244 | 209 sh |
| 3 b | 396 | 355 | | 266 | 236 sh | 211 |
| 3 c | 387 | 345 | | 266 | 236 | 210 |

Tab. 2

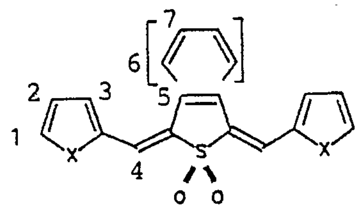

$^1H$ - NMR - Daten (in DMSO $d_6$)

| | H 1 | H 2 | H 3 | H 4 | H 5 | H 6 | H 7 |
|---|---|---|---|---|---|---|---|
| 1a | 7.91 d | 7.28 dd | 7.67 d | 7.49 s | 7.46 s | | |
| 1b[a)] | 7.1 dd | 6.23 dd | 6.83 dd | 6.89 s | 7.35 s | | |
| 1c[b)] | 7.1 dd | 6.30 dd | 6.77 dd | 6.86 s | 7.35 s | | |
| 1d | | 7.38 d | 7.51 d | 7.44 s | 7.39 s | | |
| 3a[c)] | 7.90 d | 7.25 dd | 7.87 d | 8.05 s | | 7.91 dd | 7.48dd |
| 3b[d)] | 7.09 dd | 6.20 dd | 7.34 dd | 7.47 s | | 7.86 dd | 7.36dd |
| 3c[e)] | 7.17 d | 6.31 dd | 7.17 d | 7.51 s | | 7.60 dd | 7.36dd |

a) N - $CH_3$ = 3.75 ppm
b) N - H = 11.21 ppm (Aceton $d_6$)
c) stark überlagerte Multipletts bei H1, H3 und H5
d) N - $CH_3$ = 3.82 ppm
e) N - H = 11.21 ppm

s = Singulett d = Dublett dd = Dublett eines Dubletts
Alle Angaben in ppm

Beispiel 2: Sulfoxide
(UV- und NMR-Spektraldaten sind in Tab. 3 bzw. Tab. 4 angegeben)

2.1 2,5-Bis-(2-thienylmethyliden)-2,5-dihydrothiophen-1-oxid (5)
($C_{14}H_{10}OS_3$, M = 290)

30 mmol = 3.06 g 2,5-Dihydrothiophen-1-oxid (M = 102) (4) und 70 mmol = 7.84 g = 6.5 ml Thiophen-2-aldehyd (M = 112, d = 1.2 g/cm$^3$) wurden zu einer Lösung von 0.9 g NaOH in 50 ml $H_2O$ und 12.5 ml Tetrahydrofuran gegeben. Man ließ 2 Tage rühren. Die Reinigung erfolgte durch säulenchromatographische Trennung; das Reaktionsgemisch wurde auf eine Säule Kieselgel/Diethylether aufgetragen. Der Aldehyd wurde mit Ether eluiert; das Produkt mit Essigester. Beim Abziehen des Lösungsmittels im Vakuum fiel das Produkt aus. Der Niederschlag wurde abgesaugt und getrocknet.
Ausbeute: 500 mg = 5.7 % d. Theorie
Smp.: 136 - 37° C (Zersetzung)
MS: m/e 290 ($M^+$)

2.2 1,3 Bis (arylmethyliden)-1,3-dihydroisothianaphthen-2-oxid (7)

12 mmol = 1.82 g 1,3-Dihydroisothianaphthen-2-oxid (M = 152) (6) und 26 mmol = 2.9 g = 2.4 ml Thiophen-2-aldehyd (M = 112, d = 1.2 g/cm$^3$) wurden zu einer Lösung von 1 g NaOH in 30 ml Methanol gegeben. Man ließ 3 Tage bei 40° C rühren.

aryl = 2-thienyl (7a) ($C_{18}H_{12}OS_3$, M = 340)
Aufarbeitung: Der ausgefallene Niederschlag wurde abgesaugt, mit Methanol gewaschen und aus Aceton umkristallisiert.
Ausbeute: 1.8 g = 44 % d. Theorie
Smp.: 195° C (Zersetzung)
HS: m/e 340 ($M^+$)

aryl = 2 (N-Methylpyrryl) (7b) ($C_{20}H_{18}N_2OS$, M = 334)
Aufarbeitung: Das Reaktionsgemisch wurde eingeengt und mit Kieselgel/Ether chromatographiert. Eluiert wurde mit Essigester. Das Produkt fiel beim Abziehen des Lösungsmittels in Form von violetten Kristallen an.
Ausbeute: 500 mg = 12.5 % d. Theorie
Smp.: 233° C (Zersetzung)
MS: m/e 334 ($M^+$)

Tab. 3

| | UV - Absorptionen in (nm) | | | | | |
|---|---|---|---|---|---|---|
| 5 | 412 sh | 392 | 328 | 277 | 224 sh | 212 |
| 7 a | | 361 | 330 | 273 | | |
| 7 b | | 390 | 354 | 273 | 240 | |

Tab. 4

$^1$H-NMR-Daten

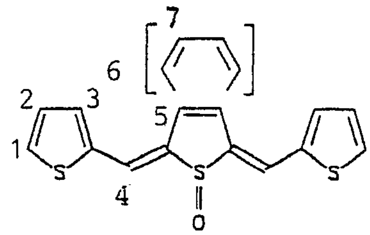

| | H 1 | H 2 | H 3 | H 4 | H 5 | H 6 | H 7 |
|---|---|---|---|---|---|---|---|
| 5 | 7.82d | 7.20dd | 7.53d | 7.51s | 7.4s | | |
| 7a | 7.88d | 7.24dd | 7.67d | 8.18s | | 7.89dd | 7.43dd |
| 7b[a)] | 7.07dd | 6.23dd | 7.17dd | 7.75s | | 7.91dd | 7.36dd |

a) N - $CH_3$ = 3.38 ppm
s = Singulett, d = Dublett, dd = Dublett
alle Angaben in ppm

Beispiele 3: "Sulfide"
(UV- und NMR-Spektraldaten sind in Tab. 5 bzw. Tab. 6 angegeben)

3.1 2,5-Bis-(2-thienylmethyliden)-2,5-dihydrothiophen (9 a)
($C_{14}H_{10}S_3$, M = 274)

Zu einer Lösung von 1.03 mmol = 300 mg 5 a (M = 292) in 30 ml trockenem Toluol und 1.25 mmol = 0.1 g = 0.1 ml Pyridin (M = 79, d = 0.978 g/cm$^3$) werden langsam 1.1 mmol = 0.19 g = 0.13 ml Phosphorigsäure-(o-phenylenester)-chlorid (M = 174.5, d = 1.46 g/cm$^3$) zugegeben. Anschließend läßt man 2 - 3 h bei Raumtemperatur rühren. Die Reaktion wird unter Luft- und Feuchtigkeitsausschluß durchgeführt. Die Umsetzung erfolgt nahezu quantitativ. Das Produkt wird säulenchromatographisch gereinigt (Kieselgel/Ether: n-Hexan 1 : 1). Nach Abziehen des Lösungsmittels erhält man ein relativ luftstabiles gelbes Pulver, das jedoch in einer $N_2$-Atmosphäre gelagert werden muß.
Ausbeute: 220 mg = 80.3 % d. Theorie
Smp.: 125 - 30$^\circ$ C (Zersetzung ohne definierten Schmelzpunkt)
MS: m/e 274 ($M^+$)

3.2 1,3-Bis-(2-thienylmethyliden)-1,3-dihydroisothianaphthen (9 b)
($C_{18}H_{12}S_3$, M = 324)

Zu einer Lösung von 3 mmol = 0.98 g 7a (M = 340) in 20 ml trockenem Toluol und 4 mmol = 0.32 g = 0.33 ml Pyridin (M = 79, d = 0.978 g/cm$^3$) werden langsam 4 mmol = 0.7 g = 0.5 ml Phosphorigsäure-(o-phenylenester)-chlorid (M = 174.5, d = 1.46 g/cm$^3$) zugegeben. Reaktionsführung und Aufarbeitung erfolgt analog zu 9 a, Kap. 3.1..
Ausbeute: 800 mg = 82.3 % d. Theorie

Smp.: 183° C
MS: m/e 324 ($M^+$)

3.3 2,5-Bis-((di-2-thienyl)-methyliden)-2,5-dihydrothiophen (9 c)
$C_{22}H_{14}S_5$, M = 438)

Zu einer Lösung von 6 mmol = 2.8 g 2,5Bis-(di-2-thienyl)hydroxymethyl)-thiophen (M = 472) (8) in 100 ml Toluol wird eine Lösung von 10 g $Na_2S_2O_4$ und 5 ml konz. Jodwasserstoffsäure in 100 ml $H_2O$ zugegeben. Nach 12-stündigem Rühren des Zweiphasen-Systems wird das Reaktionsgemisch mit $NaHCO_3$ neutralisiert und mit Ether mehrfach ausgeschüttelt. Man erhält eine orange-violette Lösung. Die Isolierung des Produkts erfolgt auf säulenchromatographischem Wege (Kieselgel/n-Hexan). Die organge-rote Lösung wird abrotiert und der rote Feststoff aus Aceton umkristallisiert.
Ausbeute: 600 mg = 23 % d. Theorie
Smp.: 157° C
MS: m/e 438 ($M^+$)

Tab. 5

UV-Daten (in Methanol)

| | UV - Absorptionen in (nm) | | | | | |
|---|---|---|---|---|---|---|
| 9a | 433 | 415 | 395 sh | 297 | 291 sh | 232 |
| 9b | 423 | 400 | 380 | 343 | 326 sh | 316 |
| | | | | 304 sh | 263 | 235 sh |
| 9c | 472 | 447 sh | 305 | 228 | | |

Tab. 6

$^1$H-NMR-Daten

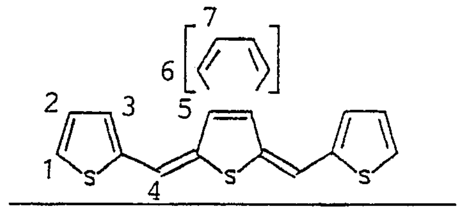

| | H 1 | H 2 | H 3 | H 4 | H 5 | H 6 | H 7 |
|---|---|---|---|---|---|---|---|
| 9a | 7.68d | 7.17dd | 7.26d | 7.24s | 7.0s | | |
| 9b | 7.73d | 7.21dd | 7.38d | 7.91s | | 8.04dd | 7.45dd |
| 9c[a)] | | 7.35 | bis | 7.03 | 6.87s | | |

a) $CDCl_3$: überlagerte Multipletts

Beispiele 4: Polymere

4.1 Die Verbindungen (1 a) und (1 b), hergestellt gemäß Beispiel 1.1 bzw. 1.2 wurden mit Nitrosyltetrafluoroborat in 1,2 Dichlorethan als Lösungsmittel polymerisiert. Erhalten wurden braune bis schwarze Niederschläge.

4.2 Die Verbindungen (1 d), hergestellt gemäß Beispiel 1.3 wurde mit n-Butyllithium in Gegenwart von $CuCl_2$ in Tetrahydrofuran polymerisiert. Erhalten wurde ein brauner Niederschlag.

4.3 Die Verbindungen (1 a) und (1 b), hergestellt gemäß Beispiel 1.1 bzw. 1.2 wurden elektrochemisch in Acetonitril in Gegenwart von Tetra-n-butylammoniumhexafluorphosphat als Leitsalz polymerisiert. Erhalten wurde ein brauner Niederschlag.

4.4 Die Verbindung (9a) gemäß Beispiel 3.1 wurde in Methylenchlorid in Gegenwart von $(n\text{-}C_4H_9)_4BF_4$ als Leitsalz elektrochemisch polymerisiert. Auf ITO (Indiumzinnoxid) - Glas als Elektrodenmaterial erhält man einen schwarz-violetten Niederschlag, der eine breite UV-Absorption mit einem Maximum bei 900 nm aufweist. Dies könnte ein Hinweis auf ein Polymer mit einer Bandstruktur, mit einem Bandgap von ungefähr 1,3 eV sein.

**Patentansprüche**

**1.** Verbindungen der Formel (I):

$$\begin{array}{c} A-C=B=C-A \\ \;\;\;|\;\;\;\;\;\;| \\ \;\;\;R'\;\;\;R'' \end{array} \qquad (I),$$

worin

**A** gleiche oder verschiedene, gegebenenfalls substituierte Heteroarylreste,

B gegebenenfalls substituierte Dihydroheteroarendiyliden-Reste oder die aus diesen Resten durch Oxidation an mindestens einem Heteroatom erhältlichen Reste,

**R'** und **R''** gleiche oder verschiedene Reste, nämlich Wasserstoffatome, $C_1$- bis $C_4$-Alkylreste oder

Reste **A** oder Cyanoreste, Halogenatome, Reste der Formel -COOR, oder -OR, worin R ein $C_1$- bis $C_4$-Alkylrest ist,
bedeuten.

**2.** Verbindungen der Formeln (IV), (V) oder (VI):

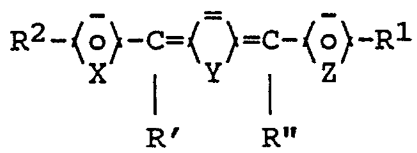

(IV),

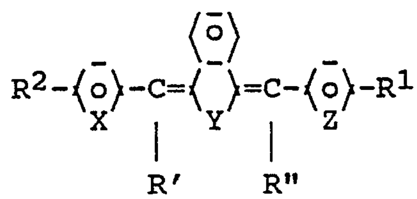

(V),

bzw.

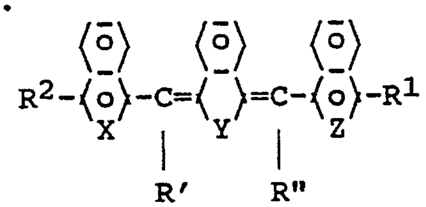

(VI),

worin bedeuten

**X** und **Z** gleiche oder verschiedene zweiwertige Reste bedeuten, nämlich Sauerstoff- oder Schwefelatome, Reste der Formel N-H oder N-R,

**R¹** und **R²** Wasserstoffatome, Halogenatome, Pseudohalogenreste, wie der Cyanorest, gegebenenfalls halogenierte $C_1$- bis $c_6$-Kohlenwasserstoffreste und gegebenenfalls halogenierte $C_1$- bis $C_6$- Kohlenwasserstoffoxireste,

**Y** ein Sauerstoff- oder Schwefelatom, ein Rest der Formel SO, $SO_2$, N-H, N-O oder N-R ist, wobei

**R** für einen $C_1$-bis $C_4$-Alkylrest steht.

**R'** und **R"** die in Anspruch 1 angegebenen Bedeutungen haben.

**3.** Verfahren zur Herstellung von Verbindungen der Formel (I) gemäß Anspruch 1 durch Umsetzung von Dihydroheteroarenen der Formel (VII)

$BH_4$ (VII),

mit Verbindungen der Formeln (VIII) und (IX)

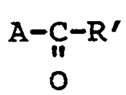

(VIII) bzw.

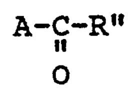

(IX)

wobei in den obigen Formeln (VII), (VIII) und (IX) **A**, **B**, **R'** und **R"** die in Anspruch 1 unter Formel (I) angegebenen Bedeutungen haben.

**4.** Verfahren zur Herstellung von Verbindungen der Formeln (IV), (V) oder (VI) gemäß Anspruch 2, wobei Verbindungen der Formel (X)

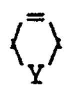 (X),

und/oder Verbindungen der Formel (XI)

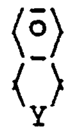 (XI),

und/oder Verbindungen der Formel (XXVII)

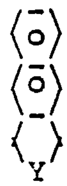 (XXVII);

und/oder Verbindungen der Formel (XXVIII)

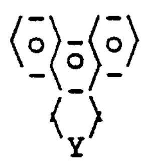 (XXVIII);

mit Verbindungen der Formeln (XII), (XIII), (XIV) beziehungsweise (XV)

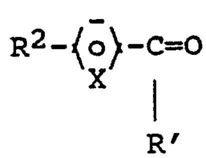 (XII), 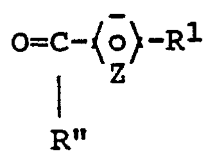 (XIII),

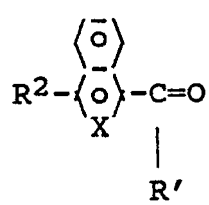 (XIV) bzw. 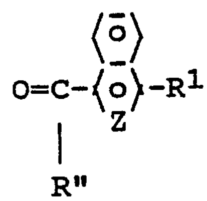 (XV),

mit der Maßgabe, daß, falls Y im Produkt gemäß Formeln (IV), (V) oder (VI) ein Schwefelatom bedeuten soll, vorzugsweise Verbindungen der Formel (X), (XI), (XXVII) und/oder (XXVIII) eingesetzt werden, für die Y einen Rest der Formel SO bedeutet, und das als Reaktionsprodukt erhaltene Sulfoxid anschließend reduziert wird.

5. Verfahren nach Anspruch 3 oder 4, wobei Verbindungen der Formeln (VIII), (IX), (XII), (XIII), (IV) beziehungsweise (XV) eingesetzt werden, für die R' und R" jeweils Wasserstoffatome sowie vorzugsweise $R^1$ und $R^2$ die gleiche Bedeutung haben.

6. Verfahren nach einem der Ansprüche 3, 4 oder 5, welches in Gegenwart einer Base durchgeführt wird.

7. Verfahren zur Herstellung von Verbindungen der Formel (I) gemäß Anspruch 1, wobei Verbindungen (VIII) und/oder (IX) gemäß Anspruch 3 mit Lithiumsalzen der den Resten **B** in Formel (I) entsprechenden Heteroarenen zur Reaktion gebracht werden und die so erhaltenen Produkte anschließend reduziert werden.

8. Verwendung der Verbindungen gemäß Anspruch 1 zur Herstellung von Polymeren.

9. Polymere der Formel (XIX)

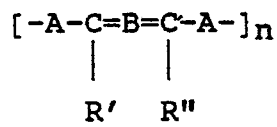

(XIX)

und Komplexe hiervon mit Dotierungsmitteln,
worin

**n** eine ganze Zahl bedeutet und

**A**, **B**, **R'** und **R"** die in Anspruch 1 angegebenen Bedeutungen haben.

10. Verwendung der Polymere gemäß Anspruch 9 als elektrische Leiter oder Halbleiter, als antistatische Materialien, für Beschichtungen von optischen Artikeln, in Wirkstoff enthaltenden Präparaten, als Absorbentien für Chemikalien, als oder in Katalysatoren oder deren Vorläufern.